# EUROPEAN PATENT SPECIFICATION

(11) **EP 3 919 120 B1**
(45) Date of publication and mention of the grant of the patent: **30.04.2025**
(21) Application number: 19913187.1
(22) Date of filing: 18.04.2019
(51) Int. Cl.: A61N 1/44, H01T 23/00, A61H 33/14, A61L 9/22, F24F 11/00

(54) **METHOD AND APPARATUS FOR REALIZING AIR CHARGED PARTICLE WAVE WITH REQUIRED FREQUENCY**
VERFAHREN UND VORRICHTUNG ZUR ERZEUGUNG EINER LUFTGELADENEN TEILCHENWELLE MIT ERFORDERLICHER FREQUENZ
PROCÉDÉ ET APPAREIL POUR RÉALISER UNE ONDE DE PARTICULES CHARGÉES DANS L'AIR AVEC UNE FRÉQUENCE REQUISE

(30) Priority: 02.02.2019 CN 201910106917
(43) Date of publication of application: 08.12.2021
(73) Proprietor: Liu, Yanbing, Jinan, Shandong 250000 (CN)
(72) Inventor: LIU, Yanbing, Jinan, Shandong 250000 (CN); LIU, Qijia, Richmond, BC V7C 2N8 (CA); LIU, Qirui, Richmond, BC V7C 2N8 (CA)
(74) Representative: Germain Maureau
(86) International application number: PCT/CN2019/083285
(87) International publication number: WO 2020/155412

(56) References cited:
- EP-A1- 2 527 002
- CN-A- 1 079 915
- CN-A- 109 771 825
- JP-A- 2005 328 904
- US-A1- 2015 108 364
- US-A1- 2016 367 712

## Description

### TECHNICAL FIELD

The present disclosure relates to a method and apparatus for realizing an air charged particle wave with a required frequency.

### BACKGROUND

Charged particles refer to charged microparticles in various forms, and can be positively-charged positive ions or negatively-charged negative ions and charged spatial inherent particles and the like. Collision or contact of the charged particles with a certain energy, a certain conversion frequency and movement direction is conducive to health and physical therapy and healthcare.

Negative air ions are widely applied charged particles and are usually generated by ionizing air by means of a high voltage or a strong ray, which are conducive to physical and psychological health of the human body and primarily improve the function of the human by means of a nervous system and blood circulation of a human to promote health. The negative air ions can make a cerebral cortex inhibiting process of a human strengthen and adjust the function of the cerebral cortex, such that the negative air ions can play roles of calming, hypnotizing and reducing blood pressure.

In an existing charged particle generation system or apparatus, the charged particles are emitted randomly. Only the amount of the charged particles is emphasized without intentionality and directionality. The movement direction, density, frequency and direction cannot be regulated and controlled, and moreover, the charged particles are relatively low in energy and poor in physical therapy effect.

US2016367712A1 discloses a sanitizer for sanitizing various surfaces. JP2005328904A discloses an ion generator capable of generating positive ions and negative ions in air, as well as an air conditioner using the same. EP2527002A1 discloses a method and a facial care apparatus for increasing a moisture content of a skin and skin elasticity by ions. US2015108364A1 discloses a charged particle emission device and an air-blowing device.

### SUMMARY

The present invention provides a method and apparatus for realizing an air charged particle wave with a required frequency as defined in claims 1 and 3 respectively.

The method and structure are reasonable in design. Charged particles propagate in the air in a form of a charged particle wave matched with various physiological waveforms of a human body, and the charged particles with the required frequency can be regulated and output under different application scenes, the obtained charged particle wave with different frequencies and charge amounts is regulated and controlled physiologically correspondingly to meet an application requirement of treating and improving health. The method and apparatus are wide in application range, and solve problems existing in the prior art.

The technical solution adopted by the present disclosure to solve the technical problems is
a method provided according to claim 1 and an apparatus for realizing an air charged particle wave with a required frequency provided according to claim 3.

By adopting the above structure, by matching the charged particle generation unit with the waveform control unit, different charged particle wave output frequencies are modulated to be suitable for different application scenes. In a real life, brain waves acting on the human body are mainly divided into α waves, β waves, θ waves, δ waves and Schumann waves, wherein an oscillation frequency of the α waves is 8-13 Hz, an oscillation frequency of the β waves is 13-14 Hz, an oscillation frequency of the θ waves is 4-7 Hz, an oscillation frequency of the δ waves is 0-4 Hz, and an oscillation frequency of the Schumann waves is 7.83 Hz. The brain waves correspond to different function states of the human body. The apparatus outputs the charged particle waves in different waveforms to assist a user according to the function state of the user body. The charged particle waves with different frequencies are obtained according to the output waveforms in the control unit by means of the execution module, and the execution module can be a power supply, a fan or an air channel. By amplifying the output waveforms pre-stored in the control unit by regulating an amplifier chip, the output waveforms are more precise, such that the apparatus has the advantages of large application range and high practicality.

### BRIEF DESCRIPTION OF THE DRAWINGS

Fig. 1 is a flow schematic diagram of Embodiment 1 of the present disclosure.
Fig. 2 is a flow schematic diagram of Embodiment 2 of the present disclosure.

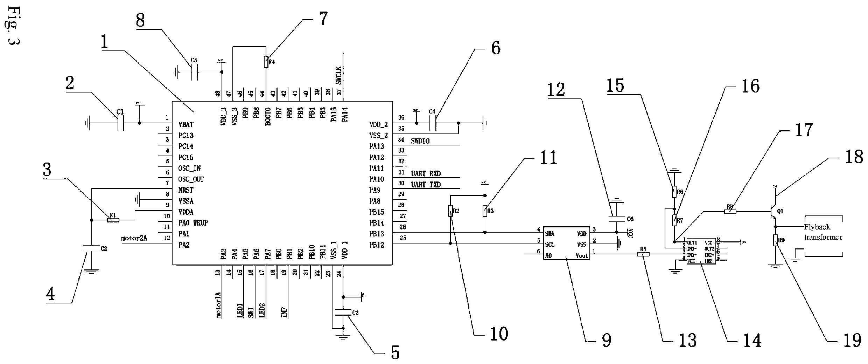

Fig. 3 is an electrical schematic diagram of the present disclosure.

In the drawings, 1, microcontroller, 2, first capacitor, 3, first resistor, 4, second capacitor, 5, third capacitor, 6, fourth capacitor, 7, fourth resistor, 8, fifth capacitor, 9, digital-to-analogue conversion chip, 10, second resistor, 11, third resistor, 12, sixth capacitor, 13, fifth resistor, 14, amplifier chip, 15, sixth resistor, 16, seventh resistor, 17, eighth resistor, 18, triode, and 19, ninth resistor.

### DETAILED DESCRIPTION

In order to describe the technical features of the solution clearly, detailed description will be made to the present disclosure below by means of specific embodiments in combination with drawings thereof.

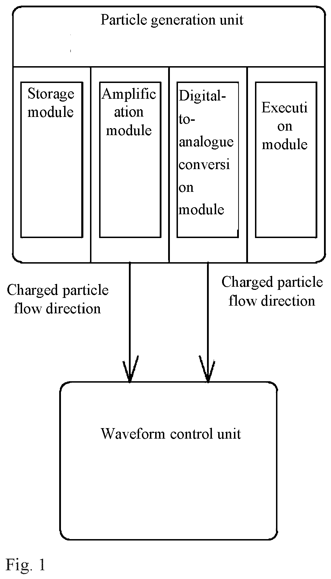

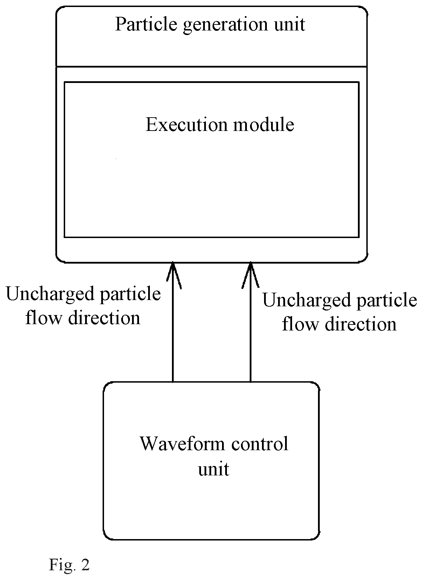

As shown in the Figs. 1-2, an apparatus for realizing an air charged particle wave with a required frequency, the apparatus including:
a charged particle generation unit, wherein the charged particle generation unit is configured to generate charged particles;
a waveform control unit, wherein the waveform control unit is configured to change the density, frequency, direction and energy of the charged particles generated by the charged particle generation unit, such that the particles form a charged particle wave in a predetermined waveform.

The charged particle generation unit includes:
a storage module configured to pre-store a set charged particle wave by the charged particle generation unit,
an amplification module configured to amplify the charged particle waveform by the charged particle generation unit,
a digital-to-analogue conversion module configured to convert the charged particle waveform by the charged particle generation unit, and
an execution module configured to generate charged particles by the charged particle generation unit according to the charged particle waveform.

The waveform control unit is a fan, configured to change an output amount of the charged particles by regulating an air speed of the fan.

The waveform control unit is an air channel, and the air channel is provided with a closing mechanism, configured to change an output amount of the charged particles by regulating a dimension of the air channel.

A method for realizing an air charged particle wave with a required frequency, includes the following step:
controlling, by a waveform control unit, a charged particle generation unit, so that the charged particle generation unit generates a charged particle wave at a preset frequency.

Controlling, by the waveform control unit, the charged particle generation unit includes the following steps:
reading, by the charged particle generation unit, a charged particle waveform pre-stored in the charged particle generation unit according to a user requirement;
carrying out amplification treatment on the charged particle waveform;
converting the charged particle waveform;
generating the charged particle wave according to the charged particle waveform and transmitting the generated charged particle wave to the waveform control unit to enable the waveform control unit to regulate the charged particle wave.

Controlling, by the waveform control unit, the charged particle generation unit includes the following step:
transmitting, by the waveform control unit, particles to the charged particle generation unit according to the preset frequency to enable the charged particle generation unit to process the charged particles so as to form the charged particle wave with a specific frequency.

In an actual embodiment, an apparatus for realizing an air charged particle wave with a required frequency includes a microcontroller, an executor and a charged particle generator. The microcontroller is connected with the executor, and the executor is connected with the charged particle generator; a model number of the microcontroller is an STM32 serial chip, the microcontroller is provided with 48 pins, a first pin is connected with a first capacitor, and the first capacitor is earthed; a seventh pin is connected with a first resistor, the first resistor is connected with a ninth pin, the seventh pin is connected with a second capacitor, and the second capacitor is earthed; a twenty-fourth pin is connected with a third capacitor, the third capacitor is connected with a twenty-third pin and is earthed respectively; a thirty-sixth pin is connected with a fourth capacitor, and the fourth capacitor is connected with a thirty-fifth pin and is earthed, respectively; a forty-fourth pin is connected with a fourth resistor, and the fourth resistor is connected with a forty-seventh pin; a forty-eighth pin is connected with a fifth capacitor, and the fifth capacitor is earthed; the first pin, the twenty-fourth pin, the thirty-sixth pin and the forty-eighth pin are connected with a power supply end respectively; a twelfth pin and a thirteen pin of the microcontroller are fan pins, a fifteen pin and a seventh pin of the microcontroller are LED indicator light pins, a sixteenth pin of the microcontroller is a control button pin, a nineteenth pin of the microcontroller is a remote controller pin, a thirtieth pin and a thirty-first pin of the microcontroller are serial port pins, a thirty-fourth pin and a thirty-seventh pin of the microcontroller are program burning pins, and the microcontroller is connected with a memory through the thirty-fourth pin and the thirty-seventh pin, such that the memory transmits the pre-stored charged particle waveform to the microcontroller.

The microcontroller is further connected with a digital-to-analogue conversion chip, the digital-to-analogue conversion chip is provided with six pins, the microcontroller is connected with a fourth pin and a fifth pin on the digital-to-analogue conversion chip via a twenty-fifth pin and a twenty-sixth pin, the twenty-fifth pin of the microcontroller is connected with a second resistor, the second resistor is connected with a third resistor, the third resistor is connected with the twenty-sixth pin, and the twenty-fifth pin and the twenty-sixth pin are connected with the power supply end respectively.

The model number of the digital-to-analogue conversion chip is one of DAC5571, DAC5573 or DAC5574. A second pin of the digital-to-analogue conversion chip is earthed, a third pin of the digital-to-analogue conversion chip is connected with a sixth capacitor, the sixth capacitor is earthed, and the third pin is further connected with the power supply end; and a first pin of the digital-to-analogue conversion chip is connected with a fifth resistor, and the fifth resistor is connected with an amplifier chip.

The model number of the amplifier chip is LM386, the amplifier chip is provided with eight pins, an eighth pin is connected with a power supply, and a fourth pin is earthed; a third pin is connected with the fifth resistor, and the fifth resistor is connected with the first pin of the digital-to-analogue conversion chip; a second pin is connected with a sixth resistor, the sixth resistor is earthed, the second pin is connected with a seventh resistor and the seventh resistor is connected with a first pin; the first pin is connected with an eight resistor, the eighth resistor is connected with a triode, the triode is connected with the particle generator, and the particle generator is earthed; the triode is a NPN type triode, the first pin of the amplifier chip is connected with the eighth resistor, the eighth resistor is connected with a base electrode of the triode, a collecting electrode of the triode is connected with the power supply, an emitting electrode of the triode is connected with the particle generator, the emitting electrode of the triode is connected with a ninth resistor, and the ninth resistor is earthed.

The charged particle generator is a flyback transformer, and the model number of the flyback transformer is SKR1W12VDC1. The executor includes a voltage source, a fan or an air channel. The executor is further provided with a directional circulation dredging apparatus. The output waveform of the charged particle wave can also be adjusted by turning on and off the directional circulation dredging apparatus, thereby achieving an effect of controlling the charged particle wave.

A voltage value of the power supply end is 3.3 V and a voltage value of the power supply is 15 V.

During use, the microprocessor is connected with the executor, and a control program in the memory is input into the microcontroller 1 via the program burning pins on a microcontroller 1, such that the microcontroller 1 acts to be matched with a peripheral circuit on the microcontroller 1, and different particle wave output waveforms can be output in different application scenes according to a requirement of a user. Compared with an existing charged particle preparation apparatus, the apparatus of the present disclosure has the advantages that the charged particles are output in the form of pulse and charged particle waves, such that it can be convenient to adjust correspondingly according to the requirement of the user, and therefore, the apparatus can be suitable for various different environments and occasions; four bran waves acting on the human body will appear in different function states of the human body, for example, the α brain wave appears in initial sleep or initial awakening, and at the moment, the body function of the user can be in a relaxed state and has conscious awareness; the β wave will appear when the user is awake, and at the moment, the body function of the user is in a working state and attention is focused; the δ wave and the θ wave will appear when the user is asleep, and at the moment, the body function of the user is in a rest state. According to the principle, the apparatus can output particle waves of different waveforms to assist the user according to the body state of the user, such that the user can acquire higher-quality sleep and a more efficient working state; in many frequencies, there is still a Schumann wave with the frequency of 7.83 Hz, which is conducive to the mind and body of the user, and the apparatus can also emit the particle waves with this frequency, which is equivalent to 'charge' the body of the user. In a word, the apparatus can provide beneficial particle waves to the body of the user, such that the user is in a good working state or rest state; the waveform control unit can be one of the fan and the air channel. When the executor is the fan, the microcontroller regulates the air speed of the fan according to a specific frequency, such that the output particle waves form the specific waveforms; when the executor is the air channel, the microcontroller changes the output amount of the particles by regulating the dimension of the air channel, so as to form the specific waveform of the particle wave; the directional circulation dredging apparatus connected to the executor can transmit charges of the human body to the ground, such that the physical therapy effect of the charged particle wave is improved. The charged particle flow directions of the charged particle generation unit and the waveform control unit have two different modes. One of the modes is as follows: the charged particle generation unit processes the particles according to the specific frequency, such that the particles are charged, and the waveform control unit inspects the charged particles to form the charged particle wave needed by the user; the other mode is as follows: the waveform control unit regulates the content of the particles input into the charged particle generation unit, such that the particles input into the charged particle generation unit are processed according to the specific waveform to charge the particles and form the charged particle wave; the microcontroller 1 is further connected with the digital-to-analogue conversion chip 9 and the amplifier chip 14, such that a signal transmitted to the flyback transformer is more precise, the action error of the flyback transformer is reduced, and therefore, the charged particle waveform is free of a distorting phenomenon. In the apparatus, a waveform generation module can be arranged at one side of a power supply module or can be arranged in a high voltage generation module, which can be selected voluntarily according to the requirement of the user; the fan pin on the microcontroller 1 can be connected with a function assembly such as a heat dissipating fan to dissipate heat of each module in the apparatus. The button pin and the remote controller pin make the user's operation more convenient, the serial port pins can be connected with a terminal apparatus, and the user can detect the microcontroller in real time to prevent malfunction and chaos phenomena of the apparatus. The model number of the microcontroller 1 is the STM32 serial chip which is low in cost and good in universality. The model number of the flyback transformer is SKR1W12VDC1, the flyback transformer is excellent in performance and can be suitable for various environments and occasions. Under a common condition, the charged particles are all charged negative ions, among which negative air ions are widely applied, which facilitates the physical and psychological health of the human body. The negative air ions are output outward in a form of particle wave, and the apparatus is convenient to adjust, large in application range and high in practicality. The charged particle waves with different frequencies obtained by changing the output waveforms by the microcontroller 1 are applied to different environments, such that the control unit emits the charged particles with the specific waveforms (frequencies). The directional circulation dredging apparatus is additionally provided with a control module, which can achieve the effect of controlling the waveform of the charged particles. In a word, the apparatus of the present disclosure is wide in application field and can be applied to the fields of medicine, military project, environmental protection and the like.

The scope of the present invention is deined by the appended claims.

## Claims

1. A method for realizing an air charged particle wave with a required frequency, comprising the following step:
controlling, by a waveform control unit, a charged particle generation unit, so that the charged particle generation unit generates a charged particle wave at a preset frequency;
the waveform control unit outputting particle waves of different waveforms to assist a user;
the waveform control unit being a fan or an air channel,
the fan being configured to regulate and control to change an output amount of the charged particles by regulating an air speed of the fan, such that the output particle waves form the specific waveforms;
the air channel being provided with a closing mechanism and configured to change an output amount of the charged particles by regulating a dimension of the air channel, so as to form the specific waveform of the particle wave;
**characterized in that** controlling, by the waveform control unit, the charged particle generation unit comprises the following steps:
reading, by the charged particle generation unit, a charged particle waveform pre-stored in the charged particle generation unit according to a user requirement;
carrying out amplification treatment on the charged particle waveform;
converting the charged particle waveform; and
generating the charged particle wave according to the charged particle waveform and transmitting the generated charged particle wave to the waveform control unit to enable the waveform control unit to regulate the charged particle wave;
wherein the specific waveforms correspond to α waves, β waves, θ waves, δ waves, and Schumann waves.

2. The method according to claim 1, wherein controlling, by the waveform control unit, the charged particle generation unit comprises the following step:
transmitting, by the waveform control unit, a specific regulating and controlling wave to the charged particle generation unit according to the preset frequency to enable the charged particle generation unit to regulate and control and process particles so as to form the charged particle wave with a specific frequency.

3. An apparatus for realizing an air charged particle wave with a required frequency, comprising:
a charged particle generation unit, wherein the charged particle generation unit is configured to generate charged particles; and
a waveform control unit, wherein the waveform control unit is configured to regulate and control the density and movement direction of the charged particles generated by the charged particle generation unit, such that the charged particles form a charged particle wave moving in a predetermined waveform;
the waveform control unit being configured to output particle waves of different waveforms to assist a user;
the waveform control unit being a fan or an air channel,
the fan being configured to regulate and control to change an output amount of the charged particles by regulating an air speed of the fan, such that the output particle waves form the specific waveforms;
the air channel being provided with a closing mechanism and configured to change an output amount of the charged particles by regulating a dimension of the air channel, so as to form the specific waveform of the particle wave;
**characterized in that** the charged particle generation unit comprises:
a storage module configured to pre-store a set charged particle wave by the charged particle generation unit;
an amplification module configured to regulate and control and amplify the charged particle waveform by the charged particle generation unit;
a digital-to-analogue conversion module configured to convert the charged particle waveform by the charged particle generation unit; and
an execution module configured to generate charged particles by the charged particle generation unit according to the particle waveform,
wherein the specific waveforms correspond to α waves, β waves, θ waves, δ waves, and Schumann waves.

## Patentansprüche

1. Verfahren zur Realisierung einer luftgeladenen Teilchenwelle mit einer erforderlichen Frequenz, das den folgenden Schritt umfasst:
Steuern einer Erzeugungseinheit für geladene Teilchen durch eine Wellenform-Steuereinheit, so dass die Erzeugungseinheit für geladene Teilchen eine geladene Teilchenwelle mit einer voreingestellten Frequenz erzeugt;
wobei die Wellenform-Steuereinheit die Teilchenwellen verschiedener Wellenformen ausgibt, um einen Benutzer zu unterstützen;
wobei die Wellenform-Steuereinheit ein Gebläse oder ein Luftkanal ist,
wobei das Gebläse so konfiguriert ist, dass es eine Ausgangsmenge der geladenen Teilchen durch Regulieren einer Luftgeschwindigkeit des Gebläses reguliert und steuert, so dass die Ausgangsteilchenwellen die spezifischen Wellenformen bilden;
wobei der Luftkanal mit einem Schließmechanismus versehen und so konfiguriert ist, dass er eine Ausgangsmenge der geladenen Teilchen durch Regulieren einer Abmessung des Luftkanals ändert, so dass er die spezifische Wellenform der Teilchenwelle bildet;
**dadurch gekennzeichnet, dass** das Steuern der Erzeugungseinheit für geladene Teilchen durch die Wellenform-Steuereinheit die folgenden Schritte umfasst:
Lesen einer in der Erzeugungseinheit für geladene Teilchen vorab gespeicherten Wellenform geladener Teilchen durch die Erzeugungseinheit für geladene Teilchen gemäß einer Benutzeranforderung;
Durchführen einer Verstärkungsbehandlung auf der Wellenform geladener Teilchen;
Konvertieren der Wellenform geladener Teilchen; und
Erzeugen der geladenen Teilchenwelle entsprechend der Wellenform geladener Teilchen und Übertragen der erzeugten geladenen Teilchenwelle an die Wellenform-Steuereinheit, um es der Wellenform-Steuereinheit zu ermöglichen, die geladene Teilchenwelle zu regulieren;
wobei die spezifischen Wellenformen α-Wellen, β-Wellen, θ-Wellen, δ-Wellen und Schumann-Wellen entsprechen.

2. Verfahren nach Anspruch 1, wobei das Steuern der Erzeugungseinheit für geladene Teilchen durch die Wellenform-Steuereinheit den folgenden Schritt umfasst:
Übertragen einer spezifischen Regulierungs- und Steuerwelle durch die Wellenform-Steuereinheit an die Erzeugungseinheit für geladene Teilchen gemäß der voreingestellten Frequenz, um es der Erzeugungseinheit für geladene Teilchen zu ermöglichen, Teilchen so zu regulieren und zu steuern und zu verarbeiten, dass die geladene Teilchenwelle mit einer bestimmten Frequenz gebildet wird.

3. Vorrichtung zur Realisierung einer luftgeladenen Teilchenwelle mit einer erforderlichen Frequenz, die Folgendes umfasst:
eine Erzeugungseinheit für geladene Teilchen, wobei die Erzeugungseinheit für geladene Teilchen so konfiguriert ist, dass sie geladene Teilchen erzeugt; und
eine Wellenform-Steuereinheit, wobei die Wellenform-Steuereinheit so konfiguriert ist, dass sie die Dichte und Bewegungsrichtung der geladenen Teilchen, die von der Erzeugungseinheit für geladene Teilchen erzeugt werden, reguliert und steuert, so dass die geladenen Teilchen eine geladene Teilchenwelle bilden, die sich in einer vorbestimmten Wellenform bewegt;
wobei die Wellenform-Steuereinheit so konfiguriert ist, dass sie Teilchenwellen verschiedener Wellenformen ausgibt, um einen Benutzer zu unterstützen;
wobei die Wellenform-Steuereinheit ein Gebläse oder ein Luftkanal ist,
wobei das Gebläse so konfiguriert ist, dass es eine Ausgangsmenge der geladenen Teilchen durch Regulieren einer Luftgeschwindigkeit des Gebläses reguliert und steuert, so dass die Ausgangsteilchenwellen die spezifischen Wellenformen bilden;
wobei der Luftkanal mit einem Schließmechanismus versehen und so konfiguriert ist, dass er eine Ausgangsmenge der geladenen Teilchen durch Regulieren einer Abmessung des Luftkanals ändert, so dass er die spezifische Wellenform der Teilchenwelle bildet;
**dadurch gekennzeichnet, dass** die Erzeugungseinheit für geladene Teilchen Folgendes umfasst:
ein Speichermodul, das so konfiguriert ist, dass es eine eingestellte geladene Teilchenwelle von der Erzeugungseinheit für geladene Teilchen vorab speichert;
ein Verstärkungsmodul, das so konfiguriert ist, dass es die Wellenform geladener Teilchen durch die Erzeugungseinheit für geladene Teilchen reguliert und steuert und verstärkt;
ein Digital-Analog-Umwandlungsmodul, das so konfiguriert ist, dass es die Wellenform geladener Teilchen durch die Erzeugungseinheit für geladene Teilchen umwandelt; und
ein Ausführungsmodul, das so konfiguriert ist, dass es geladene Teilchen durch die Erzeugungseinheit für geladene Teilchen gemäß der Teilchenwellenform erzeugt,
wobei die spezifischen Wellenformen α-Wellen, β-Wellen, θ-Wellen, δ-Wellen und Schumann-Wellen entsprechen.

## Revendications

1. Procédé de réalisation d'une onde de particules chargées dans l'air avec une fréquence requise, comprenant l'étape suivante :
de commande, par une unité de commande de forme d'onde, d'une unité de génération de particules chargées, de sorte que l'unité de génération de particules chargées génère une onde de particules chargées à une fréquence prédéfinie ;
l'unité de commande de forme d'onde délivrant en sortie des ondes de particules de différentes formes d'onde pour aider un utilisateur ;
l'unité de commande de forme d'onde étant un ventilateur ou un canal d'air,
le ventilateur étant configuré pour réguler et effectuer une commande afin de modifier une quantité de sortie des particules chargées en régulant la vitesse d'air du ventilateur, de sorte que les ondes de particules de sortie forment les formes d'onde spécifiques ;
le canal d'air étant muni d'un mécanisme de fermeture et configuré pour modifier une quantité de sortie des particules chargées en régulant la dimension du canal d'air, de manière à former la forme d'onde spécifique de l'onde de particules ;
**caractérisé en ce que** la commande, par l'unité de commande de forme d'onde, de l'unité de génération de particules chargées comprend les étapes suivantes :
de lecture, par l'unité de génération de particules chargées, d'une forme d'onde de particules chargées pré-stockée dans l'unité de génération de particules chargées selon les besoins de l'utilisateur ;
de réalisation d'un traitement d'amplification sur la forme d'onde de particules chargées ;
de conversion de la forme d'onde de particules chargées ; et
de génération de l'onde de particules chargées selon la forme d'onde de particules chargées et de transmission de l'onde de particules chargées générée à l'unité de commande de forme d'onde pour permettre à l'unité de commande de forme d'onde de réguler l'onde de particules chargées ;
dans lequel les formes d'onde spécifiques correspondent aux ondes α, aux ondes β, aux ondes θ, aux ondes δ et aux ondes Schumann.

2. Procédé selon la revendication 1, dans lequel la commande, par l'unité de commande de forme d'onde, de l'unité de génération de particules chargées comprend l'étape suivante :
de transmission, par l'unité de commande de forme d'onde, d'une onde de régulation et de commande spécifique à l'unité de génération de particules chargées selon la fréquence prédéfinie pour permettre à l'unité de génération de particules chargées de réguler, de commander et de traiter les particules afin de former l'onde de particules chargées avec une fréquence spécifique.

3. Appareil de réalisation d'une onde de particules chargées dans l'air avec une fréquence requise, comprenant :
une unité de génération de particules chargées, où l'unité de génération de particules chargées est configurée pour générer des particules chargées ; et
une unité de commande de forme d'onde, où l'unité de commande de forme d'onde est configurée pour réguler et commander la densité et la direction de mouvement des particules chargées générées par l'unité de génération de particules chargées, de sorte que les particules chargées forment une onde de particules chargées se déplaçant selon une forme d'onde prédéterminée ;
l'unité de commande de forme d'onde étant configurée pour délivrer en sortie des ondes de particules de différentes formes d'onde pour aider un utilisateur ;
l'unité de commande de forme d'onde étant un ventilateur ou un canal d'air,
le ventilateur étant configuré pour réguler et effectuer une commande afin de modifier une quantité de sortie des particules chargées en régulant la vitesse d'air du ventilateur, de sorte que les ondes de particules de sortie forment les formes d'onde spécifiques ;
le canal d'air étant muni d'un mécanisme de fermeture et configuré pour modifier une quantité de sortie des particules chargées en régulant la dimension du canal d'air, de manière à former la forme d'onde spécifique de l'onde de particules ;
**caractérisé en ce que** l'unité de génération de particules chargées comprend :
un module de stockage configuré pour pré-stocker une onde de particules chargées définie par l'unité de génération de particules chargées ;
un module d'amplification configuré pour réguler, commander et amplifier la forme d'onde de particules chargées par l'unité de génération de particules chargées ;
un module de conversion numérique-analogique configuré pour convertir la forme d'onde de particules chargées par l'unité de génération de particules chargées ; et
un module d'exécution configuré pour générer des particules chargées par l'unité de génération de particules chargées selon la forme d'onde de particules,
dans lequel les formes d'onde spécifiques correspondent aux ondes α, aux ondes β, aux ondes θ, aux ondes δ et aux ondes Schumann.
